# EUROPEAN PATENT SPECIFICATION

(11) **EP 2 305 144 B1**
(45) Date of publication and mention of the grant of the patent: **31.10.2012**
(21) Application number: 10755733.2
(22) Date of filing: 14.01.2010
(51) Int. Cl.: A61B 19/00, A61B 18/14

(54) **ROBOT SYSTEM FOR ENDOSCOPE TREATMENT**
ROBOTERSYSTEM FÜR ENDOSKOPBEHANDLUNG
SYSTÈME ROBOTISÉ POUR TRAITEMENT PAR ENDOSCOPE

(30) Priority: 24.03.2009 JP 2009071576
(43) Date of publication of application: 06.04.2011
(73) Proprietor: OLYMPUS MEDICAL SYSTEMS CORP., Tokyo 151-0072 (JP)
(72) Inventor: NAKAMURA, Toshio, Tokyo 192-8512 (JP)
(74) Representative: Schicker, Silvia
(86) International application number: PCT/JP2010/050333
(87) International publication number: WO 2010/109932

(56) References cited:
- JP-A- 8 164 141
- JP-A- 10 094 990
- JP-A- 61 044 588
- JP-A- 2000 254 132
- JP-A- 2004 122 286
- US-A- 6 102 850
- US-A1- 2004 199 147
- US-A1- 2008 147 090

## Description

### Technical Field

The present invention relates to a robot system for endoscopic treatment that adequately maintains a gripping state of a treatment instrument that is inserted into a body cavity.

### Background Art

In general, there is known a robotic arm system for endoscopic treatment that remotely performs desired operation with respect to a treatment instrument that is inserted into an endoscope body or a forceps channel introduced in a body cavity.

In such a system, a master unit located outside includes an operation unit that is configured by a joystick or a plurality of joint members and rod members, the joint members and the rod members being alternately coupled with each other, and a master controller that converts movement of the operation unit into an electrical signal to be output as an operation signal. In a treatment instrument, a movable region at an end portion is coupled with, e.g., a pulley that is driven by a motor in a treatment instrument drive unit through a wire and the like. When this operation unit is operated, the pulley is rotated by the motor, and the wire is paid out or pulled. As a result, a biological sample (e.g., a lesioned part) can be moved in a desired direction in a gripped state, e.g., it can be gripped and pulled up by a treatment instrument inserted in a body cavity, e.g., an arm of a straight grasping forceps or a gripping unit provided at an arm end. As a representative example, there is known a multi-degree-of-freedom robotic treatment instrument having a soft portion.

### Citation List

### Pat. Document

Pat. Document 1 : Jpn. Pat. Appln. KOKAI Publication No. 2002-200091

US 2004/0199147 A1 as closest prior art discloses a manipulator to be used for medical or clinical purposes which comprises a grip portion and a driver portion. The driver portion drives the grip portion through wires guided by a plurality of pulleys. Tensions in the wires are measured and input into a controller for controlling motors driving the pulleys.

### Disclosure of Invention

### Problem to Be Solved

The multi-degree-of-freedom robotic treatment instrument described above can be driven in a desirable manner by bending the joint portions of the arm or curving the soft portion in the state where a living tissue sample is gripped by the gripping unit at the arm end.

When the gripping portion in a gripping state is moved to a desirable position by bending the joints of the arm, some of the joints may be bent and others may stretch depending upon its moving direction. As a result of the bending or stretching movement, the wire maintaining the gripping state may loosen to decrease the gripping force, and thus, the living tissue sample may fall from the gripping portion. Conversely, the wire may become tense to increase the gripping force further. It is considered that this is because the gripping force varies depending upon the degree of the bending since the wire path length structurally differs between the outer portion of the bended portion and the inner portion thereof and the friction between the wire hanged on the joint portion and the coil pipe through which the wire passes varies in the state where the soft portion and the joint portions are bent.

As treating instruments, a bipolar high-frequency treatment instrument configured to grip a living tissue by means of two movable gripping members made of two high-frequency blades to perform treatment, and a monopolar high-frequency treatment instrument configured to grip a living tissue by means of movable gripping members one of which is made of a high-frequency blade and the other of which is made of an insulator member such as a ceramic member to perform treatment are known. To enable accurate treatment by these high-frequency treating instruments, the gripping state where a living tissue to be treated is gripped with proper gripping force has to be maintained even when the gripping portion is moved.

Accordingly, an object of the present invention is to provide a robot system for endoscopic treatment which comprises a detection unit configured to detect gripping force of a gripping portion for gripping a living tissue and provided in a robotic treatment instrument with a multi-joint structure and can maintain a proper gripping state based on a detection value obtained when the gripping portion gripping a living tissue is being moved.

### Means for Solving the Problem

To achieve the above described object, the present invention provides a robot system for endoscopic treatment including: a gripping unit configured to be inserted into a body cavity along with an endoscopic apparatus wherein a desired living tissue is to be gripped by gripping members configured to move by a wire operation; a manipulator unit having at least one degree of freedom or more and including a multi-joint structure configured to bend at a desired angle or stretch wherein the gripping unit is provided at a distal end; a treatment instrument drive unit configured to perform a pulling and a paying out of wires for a gripping motion of the gripping unit and moving a region of the multi-joint structure; a gripping force detection unit configured to detect gripping force in the gripping members when the gripping unit grips the living tissue; and a control unit configured to instruct the treatment instrument drive unit on the wire operation of the gripping members so that the gripping force detected by the gripping force detection unit falls within a range of a predetermined threshold, when the gripping unit in a gripping state is moved.

### Advantages of the Invention

The robot system for the endoscopic treatment of the present invention comprises the detection unit configured to detect gripping force of the gripping portion for gripping a living tissue and provided in the robotic treatment instrument with the multi-joint structure and can maintain a proper gripping state based on a detection value obtained when the gripping portion gripping the living tissue is being moved.

The robot system for the endoscope treatment of the present invention is configured to perform control of pushing or pulling the wire configured to drive the gripping portion until the detection value obtained from the detection unit falls within a predetermined threshold range when the gripping portion is moved by the driving of the manipulator. Therefore, a change in the gripping force due to the wire path length difference and a variation in the gripping force due to the friction between the wire and the coil pipe accompanied with the movement are prevented and a slacking or unnecessary pulling force is prevented from generating in the wire, and thus, a proper gripping state of a living tissue can be maintained.

### Brief Description of Drawings

FIG. 1 is a view showing a configuration of an entire robot system for endoscopic treatment in a first embodiment according to the present invention;
FIG. 2 is a view showing a configuration for controlling each component part and a flow of a detection signal, a control signal, and others in the first embodiment;
FIG. 3 is a flowchart for explaining wire tension control processing using a wire tension sensor in the first embodiment;
FIG. 4 is a view showing a configuration of an entire robot system for endoscopic treatment in a second embodiment according to the present invention;
FIG. 5A is a view showing a structural example of a gripping unit to which a pressure sensor is provided to detect gripping force in the second embodiment;
FIG. 5B is a view showing a structural example of a gripping unit in a monopolar high-frequency treatment instrument as a first modification of the gripping unit according to the second embodiment depicted in FIG. 5A;
FIG. 5C is a view showing a structural example of a gripping unit in a bipolar high-frequency treatment instrument as a second modification of the gripping unit according to the second embodiment depicted in FIG. 5A;
FIG. 6 is a view showing a configuration for controlling each component part and a flow of a detection signal, a control signal, and others in the second embodiment; and
FIG. 7 is a flowchart for explaining wire tension control processing using a pressure sensor in the second embodiment.

Mode for Carrying Out the Invention Hereinafter, embodiments according to the present invention will be described with reference to the drawings.

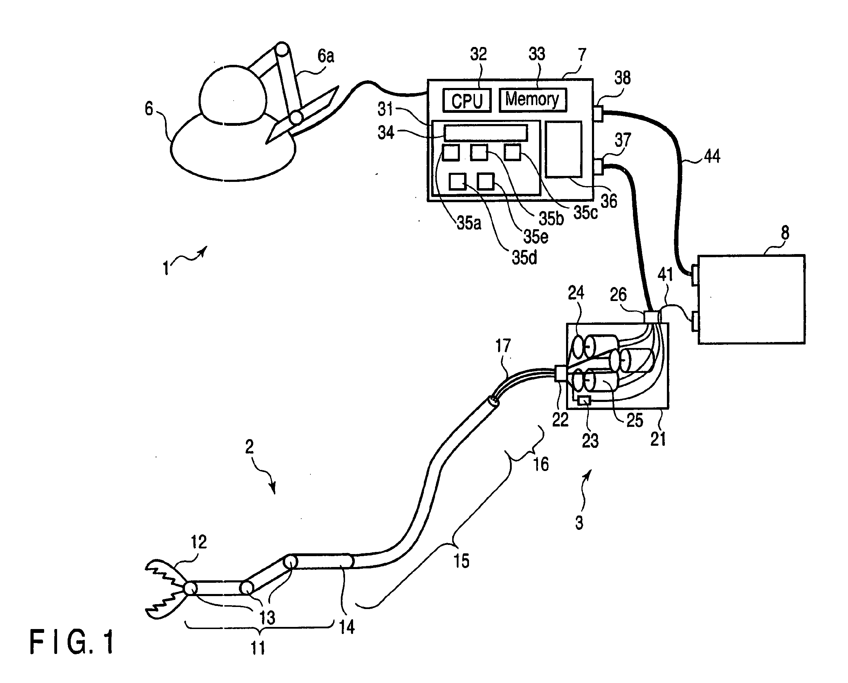

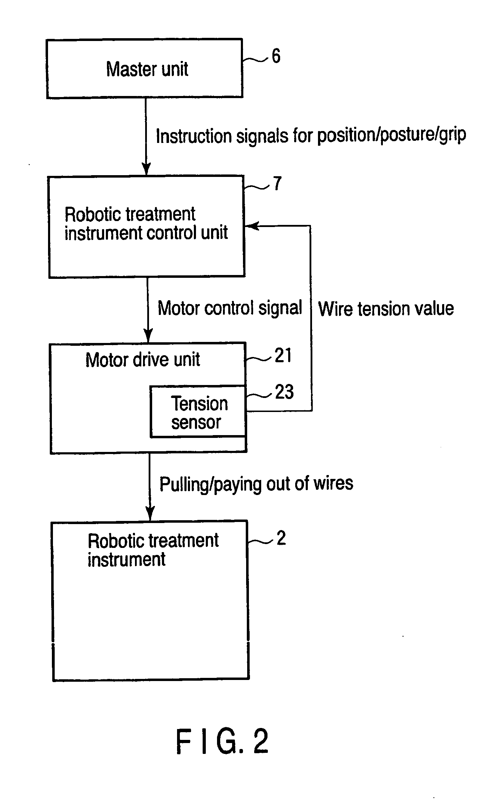

FIG. 1 is a view showing a configuration of an entire robot system for endoscopic treatment in a first embodiment according to the present invention. FIG. 2 is a view showing a configuration for effecting control in each constituent unit in the robot system for endoscopic treatment and a flow of a detection signal, a control signal, and others.

The robot system for endoscopic treatment according to this embodiment comprises a robotic treatment instrument having a multi-joint structure driven by a plurality of wires, and a wire tension sensor provided to the wires that drive a manipulator unit and a gripping unit, the wire tension sensor detecting gripping force, the robot system executing gripping control processing based on an obtained wire tension value (the gripping force).

This robot system for endoscopic treatment roughly comprises a controller 1 including a master unit 6, a robotic treatment instrument 2, and a treatment instrument drive unit 3. This robotic treatment instrument 2 is a master/slave multi-joint electric treatment instrument that follows an operation of the master unit 6 which is an instruction input device. Furthermore, the gripping unit may have a function of a bipolar high-frequency treatment instrument that holds living tissue and applies high-frequency electrical power to perform high-frequency treatment.

Although the explanation will be given as to the configuration where the master unit 6 in this embodiment issues a bend instruction for a manipulator unit 11 and an open/close instruction (gripping instruction) for a gripping unit 12, an operating portion that issues the gripping instruction may be provided separately from the master unit 6. Moreover, the master unit 6 may be configured integrally with an operation unit in the endoscope body so that one operating portion can issue an instruction for the robotic treatment instrument 2 and an instruction for the endoscope body.

The controller 1 comprises the master unit 6 which is configured to instruct a position, a posture, and gripping of the later-described robotic treatment instrument 2, a robotic treatment instrument control unit 7 that controls the robotic treatment instrument 2, and a tension sensor signal processor 8 which is configured to detect a gripping state.

The master unit 6 includes an operating portion 6a in which a plurality of joint members and rod members are alternately coupled with each other and a non-illustrated master controller that converts movement of the operating portion 6a into an electrical signal and outputs the converted signal as an operation signal. Moreover, in the operating portion 6a is provided a non-illustrated switch which is configured to instruct operations for effecting an opening/closing operation of the gripping unit 12 to grip living tissue and release the living tissue.

The robotic treatment instrument control unit 7 include an operation setting unit 31 that is configured to set various kinds of settings for the robotic treatment instrument 2; a CPU 32 that executes processing for each later-described sensor signal and various kinds of arithmetic operations, and outputs a control signal to each constituent unit in the system; a memory 33 that stores programs required for driving, obtained arithmetic operation results, and communication data; a motor driver 36 that drives and controls a motor 25 in a later-described motor drive unit 21 based on the control signal; a motor driver communication unit 37 that is configured to communicate with the later-described motor drive unit 21; and a tension sensor communication unit 38 that is configured to communicate with a later-described tension sensor signal processor 8.

The operation setting unit 31 includes operation switches 35a to 35e that is configured to set various kinds of settings and a display panel 34 that displays contents of an operation instructed by a user.

The robotic surgical instrument 2 includes the manipulator unit 11 having the multi-joint structure driven by the wires, the gripping unit 12 that is provided at an end of the manipulator unit 11 to grip living tissue (lesioned part as a treatment target), and a soft sheath portion 15 which is inserted into a non-illustrated endoscope channel and can move forward and backward. It is to be noted that the robotic treatment instrument 2 is not necessarily restricted to a usage pattern that it is inserted into the endoscope to be used, and it may be utilized separately from the endoscope. Also, the gripping unit 12 is a bipolar high-frequency treatment instrument having a configuration that two gripping members which are formed of a conductor or have opposed electrodes provided thereto, the treatment instrument opening/closing to grip living tissue and performing high-frequency treatment.

The manipulator unit 11 is provided at a distal end of the soft sheath portion 15, and is formed of at least two rod portions 14 and at least one joint portion 13 that couples the rod portions 14 with each other, resulting in the manipulator unit 11 having at least one degree of freedom. For example, the manipulator unit 11 has one degree of freedom in a vertical direction. Preferably, the manipulator unit 11 is configured in such a manner the plurality of rod portions 14 and the plurality of joint portions 13 are alternately coupled to provide different rotation surfaces of joints, so as to have six degrees of freedom in XYZ directions, a rotating direction, a yawing direction, and a pitch direction, resulting in that the manipulator unit 11 can freely bend to lift up and move the gripping unit 12. Each of the two gripping members (jaws) of the gripping unit 12 and the respective joint portions 13 are connected with wires 17 inserted in the manipulator unit 11, the sheath portion 15, and an external connecting portion 16. When these wires 17 are pulled and paid out from the external connecting portion 16, the gripping members can be opened/closed, and the respective joint portions 13 can be bent and stretched at desired angles. This opening/closing operation and the bending/stretching operation are carried out by the treatment instrument drive unit 3.

For example, if each rod portion 14 has a cylindrical shape, a connecting configuration of the rod portions 14 and the wires 17 in this embodiment is achieved by coupling each rod portion 14 with the joint points 13 at two cylinder opening ends in the horizontal direction to allow the bending operation, disposing an end of each wire 17 to each of two cylinder opening ends in the vertical direction orthogonal to the horizontal direction, and coupling the other end of the same with a manipulator (e.g., a pulley pivotally supported by a motor). When bending the rod portion 14 with respect to the joint portion 13, one of the wires 17 is pulled and the other of the wires 17 is paid out, resulting in that the rod portion 14 bends upward (or downwards) around a coupling portion between the rod portion 14 and the joint portion 13. Of course, this embodiment is not restricted to such a multi-joint structure, and a generally known multi-joint structure can be applied to this embodiment.

The treatment instrument drive unit 3 includes a motor drive unit 21 with a plurality of motors which are individually controlled. The motor drive unit 21 includes a plurality of pulley 24 coupled with the wires 17, a plurality of motors 25 which axially support the respective pulleys 24, wire tension sensors 23 which measure tensile force of respective wires 17, and a motor driver communication unit 26 which communicates with the robotic treatment instrument control unit 7.

In this embodiment, one pulley 24 is coupled with a set of wires 17 connected with the two movable gripping members of the gripping unit 12 or respective joint portion 13 through a wire coupling portion 22. Furthermore, although the description has been given on the example where a combination of the motor and the pulley is a manipulator that drives the wires 17, a combination of a servomotor and a bar-like coupling tool may also be adopted. In this case, two wires are coupled with both ends of the bar-like coupling tool and fixing a central portion of this tool to the servomotor. Moreover, electric hydrodynamic drive based on a combination of a hydraulic piston, an electric pump, and a valve can also be considered. In this case, wires are respectively coupled with the two hydraulic pistons, and the valve is opened/closed to pull/pay out the wires.

The motor drive unit 21 further includes a non-illustrated motor which rotates the robotic treatment instrument 2 on a longitudinal axis, a non-illustrated motor which moves forward and backward the robotic treatment instrument 2 (the sheath portion 15), and a non-illustrated encoder which measure a rotating angle of each motor. It is to be noted that the wire tension sensor 23 may be arranged in the wire coupling portion 22. As the wire tension sensor 23, a strain gauge that can detect a slight change in length of each wire 17 in a longitudinal axis direction is preferable. A wire tension value measured by the wire tension sensor 23 is output to the tension sensor signal processor 8 through a cable 41 and further supplied to the robotic treatment instrument control unit 7 through a cable 44.

The component parts and a flow of signals in the robot system for endoscopic treatment will now be described with reference to FIG. 2.

The master unit 6 output an instruction signal which instructs a position and a posture with respect to the manipulator unit 11, and an instruction signal which instructs gripping and releasing living tissue with respect to the gripping unit 12 to the robotic surgical instrument control unit 7 by operation of the operating portion 6a. The robotic treatment instrument control unit 7 supplies a motor control signal to the motor drive unit 21 to drive the corresponding motor 25 in response to the instruction signal received from the master unit 6. Based on this driving, the pulley 24 pivotally supported by the motor 25 rotates, and then pull and pay out the wires 17 so that movement and a change in posture of the manipulator unit 11 coupled with the wires 17 or gripping and releasing of the gripping unit 12 are carried out. When the gripping unit 12 is in a gripping state, the tension sensor 23 is allowed to detect a wire tension value of the wires 17 coupled with the gripping members. This wire tension value is fed back to the robotic treatment instrument control unit 7. Based on such feedback control, the living tissue can be held by the gripping unit with the same gripping force, or the manipulator unit 11 can be maintained in the same posture (the gripping unit is maintained at the same position).

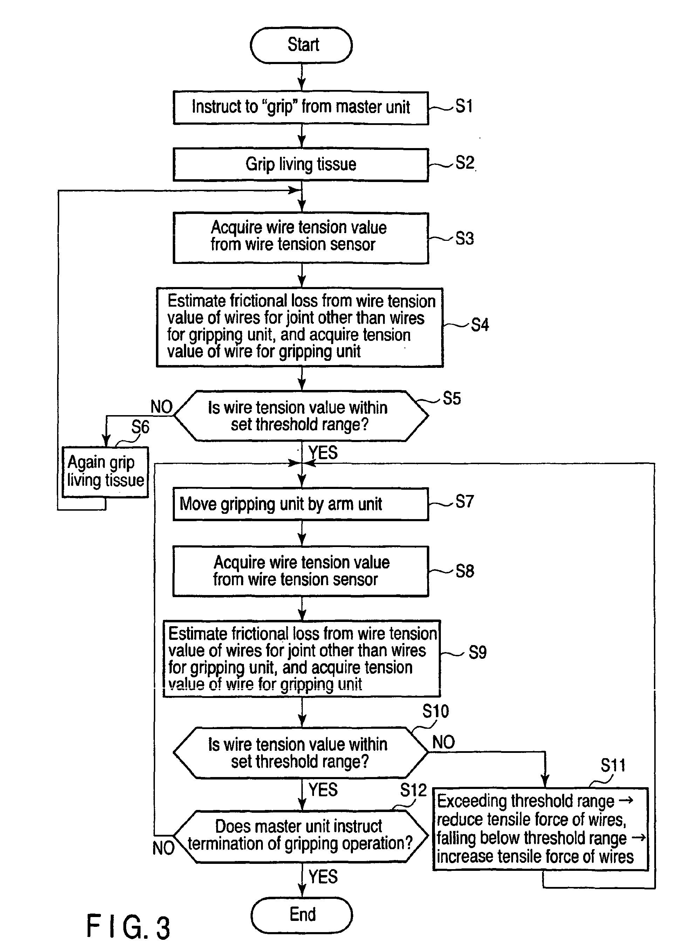

Wire tension control processing of the gripping unit 12 when movement is achieved with the manipulator unit 11 by using the wire tension sensor in the robot system for endoscopic treatment will now be described with reference to a flowchart depicted in FIG. 3.

An operator (a surgeon) operates the master unit 6 to instruct the robotic treatment instrument control unit 7 to grip desired living tissue with the gripping unit 12 inserted in a body cavity (step S1). Based on this instruction, the robotic treatment instrument control unit 7 drives the corresponding motor 25 in the motor drive unit 21 to rotate the pivotally supported pulley 24 so that the wires 17 coupled with the pulley 24 are pulled and paid out, thereby gripping the living tissue by using the gripping unit 12 (step S2).

At the time of gripping, the robotic treatment instrument control unit 7 acquires all wire tension values from the wire tension sensor 23 connected to the wires 17 (step S3). The robotic treatment instrument control unit 7 estimates a loss due to friction based on the wire tension values of the wires 17 coupled with the rod portions (or the other joint portions) excluding the wires 17 coupled with the gripping unit 12 in these wire tension values, and subtracts the loss (frictional force) caused due to the friction obtained based on the wire tension values that are utilized to drive the rod portions (the joint portions) from the wire tension value of the wires 17 that perform gripping (step S4).

It is to be noted that although the example where the tension sensor is directly disposed to the wires to measure tensile force has been described in this embodiment, the tension sensor in structural requirements of the present invention is not restricted thereto. For example, a method of acquiring tensile information from information of the utilized actuator is also an aspect as the tension sensor. For example, when a motor is used as the actuator, current measuring unit or current calculating unit functions as the tension sensor. Further, when a shaped-memory alloy (SMA) is used for the actuator, temperature information acquiring unit for the SMA functions as the tension sensor.

Step S4 will be further explained. The wire tensile force and the gripping force of the end gripping units have a correlative relationship based on a proportional relation. That is, when the wire tensile force is large, the gripping force increases in proportion to the wire tensile force. However, when the joints or the soft portions are bent, it is considered that a loss of strength due to friction of a non-illustrated coil pipe through which the wires 17 run and the wires 17 occurs. Thus, a correlation of a bending angle of any other joint portion with a wire tension value of the wires 17 that bends the joint portion and loss due to friction of the wires 17 that drive the gripping unit 12 at this moment is acquired in advance, the wire tension value of the wires 17 and loss due to friction of the wires 17 being in proportion to a magnitude of bending of each joint portion excluding the gripping unit 12. Then, when measuring the gripping force from the wire tension value, an accurate wire tension value of the wires 17 that operate the gripping unit 12 can be obtained by subtracting the loss due to the friction that is obtained by utilizing the previously acquired correlation from the wire tension value for driving the gripping unit 12. In this manner, the accurate gripping force can be obtained.

Then, the robotic treatment instrument control unit 7 compares the wire tension value obtained in step S4 with a predetermined threshold range and determines whether the wire tension value is within the threshold range (step S5). In this determination, if the wire tension value is out of the threshold range (NO), the robotic treatment instrument control unit 7 determines that the gripping unit 12 is not appropriately gripping the living tissue, controls the gripping unit 12 to again grip (step S6), and returns to step S3 to check the wire tension value. On the other hand, if the wire tension value is within the threshold range (YES), the robotic treatment instrument control unit 7 shifts to the next step. At the time of performing treatment, for example, if living tissue in a gripped lesioned part is increased due to exfoliation, the manipulator unit 11 is driven to move the gripping unit 12 while the gripping unit 12 grips the living tissue in order to visually confirm a treatment region by the surgeon (step S7). During this movement, the robotic treatment instrument control unit 7 acquires wire tension values from all the wire tension sensor 23 of the wires 17 (step S8), estimates and subtracts a loss due to friction (frictional force) from the obtained wire tension values like step S4, and acquires tensile force of the wires 17 which operate the gripping unit 12 (step S9).

Subsequently, the robotic treatment instrument control unit 7 compares the wire tension value obtained in step S9 with a predetermined threshold range and determines whether the wire tension value is within the threshold range (step S10). This determination is occasionally made in a time-sharing manner, i.e., made at appropriately set time intervals. If the wire tension value is out of the threshold range (NO) in this determination, the robotic treatment instrument control unit 7 determines that the gripping state of the gripping unit 12 is no loner adequate. The robotic treatment instrument control unit 7 drives and controls the motor 25 to reduce the wire tensile force if the wire tension value exceeds the threshold range in this determination and increase the wire tensile force if the wire tension value falls below the threshold range in the same (step S11). On the other hand, if the wire tension value is within the threshold range in the determination in step S10 (YES), the robotic treatment instrument control unit 7 determines that the gripping unit 12 keeps normally gripping the living tissue, and determines whether an instruction for termination (including temporary pause or suspension) of the gripping operation is issued from the master unit 6 in response to end of the treatment (step S12). If there is no instruction for termination of the gripping operation (NO), the robotic treatment instrument control unit 7 returns to step S7 to continue movement of the gripping unit 12. On the other hand, when there is the instruction for termination of the gripping operation (YES), the robotic treatment instrument control unit 7 terminates the series of processing.

As described above, according to the robot system for endoscopic treatment of this embodiment, the wire tension sensor is provided to the wires which drive the manipulator unit and the gripping unit of the robotic treatment instrument having the multi-joint structure. When the gripping unit is moved by driving the manipulator unit, the wires are controlled to be paid out and pulled until the wire tension value falls within the predetermined threshold range based on the wire tension value obtained from the wire tension sensor so that a normal gripping state in the gripping unit can be maintained. As a result, when the gripping unit which is in the gripping state with bending the joints in the manipulator unit is moved to a desired position, a change in gripping force due to a difference between wire path lengths or unevenness in gripping force due to friction between the wires and the coil pipe can be eliminated, slack or unnecessary pulling force is not produced in the wires, thereby maintaining the normal gripping state of the living tissue.

Furthermore, if the robotic treatment instrument is a bipolar high-frequency treatment instrument, the instrument can grip the living tissue as a treatment target with appropriate gripping force, and perform high-frequency treatment to a body mucosal membrane, e.g., sealing and cauterizing a blood vessel or a vessel bundle present inside.

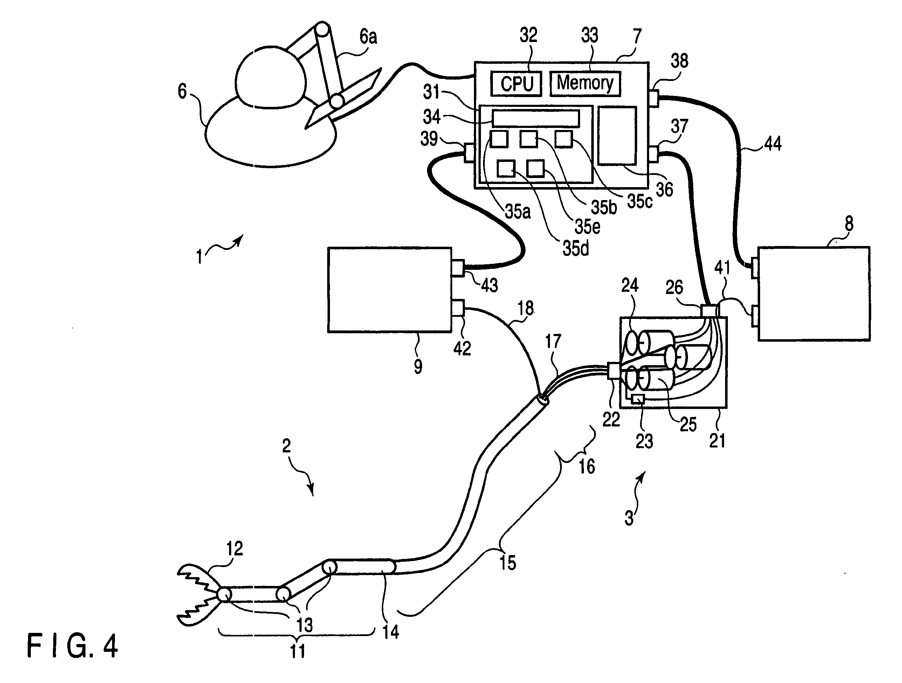

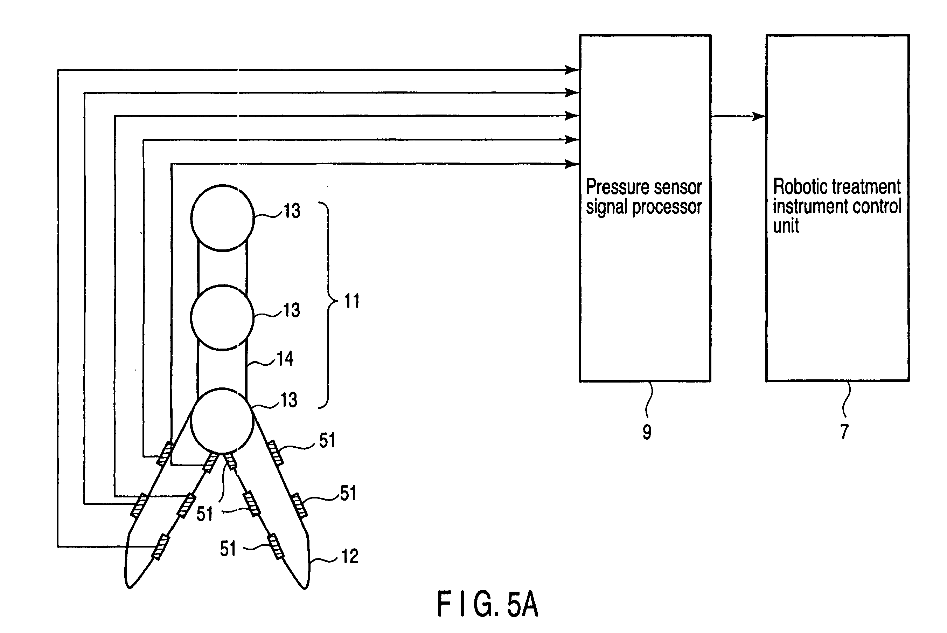

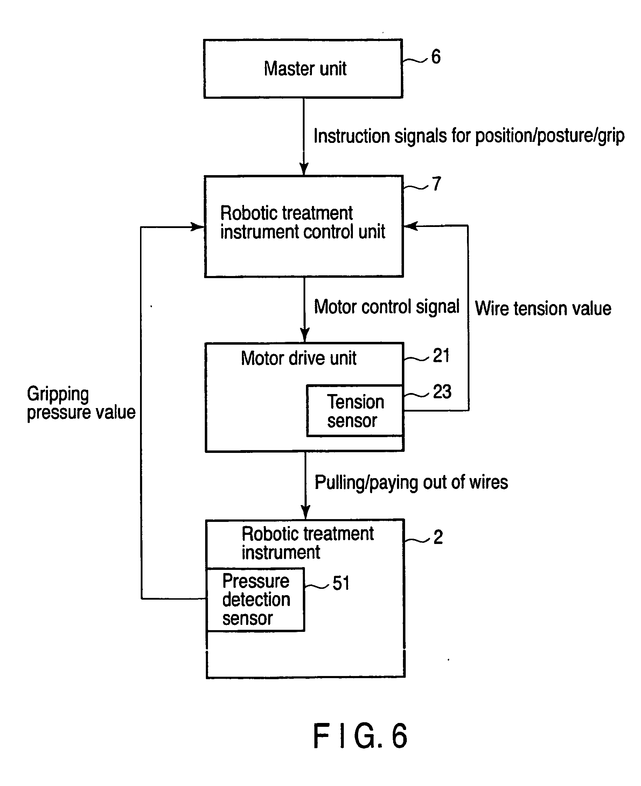

FIG. 4 is a view showing an entire configuration of the robot system for endoscopic treatment in a second embodiment according to the present invention. FIG. 5A is a view showing a structural example of a gripping unit having pressure sensors provided thereto to detect gripping force in the robot system. FIG. 6 is a view showing a configuration for controlling each component parts and a flow of a detection signal, a control signal, and others in the robot system for endoscopic treatment. Like reference numbers denote component parts in this embodiment equivalent to those shown in FIGS. 1 and 2 in the first embodiment, thereby omitting a detailed description thereof.

The robot system for endoscopic treatment according to this embodiment includes a robotic treatment instrument having a master/slave multi-joint structure which is driven by the same kinds of wires 17 as those in the first embodiment. Pressure sensors 51 configured to detect gripping force are provided to a gripping unit 12 of this robotic treatment instrument, and gripping control processing are carried out based on the gripping force value obtained when living tissue are gripped. It is to be noted that a wire tension sensor 23 is likewise provided with respect to a manipulator unit 11 in this embodiment, and the wire tension sensor does not necessarily have to be provided with respect to the gripping unit 12.

A control unit 1 includes a master unit 6, a robotic treatment instrument control unit 7, a tension sensor signal processor 8, and a pressure sensor signal processor 9 configured to detect a gripping state of the gripping unit 12. The robotic treatment instrument control unit 7 likewise includes an operation setting unit 31, a CPU 32, a memory 33, a motor driver 36, a tension sensor communication unit 38, a pressure sensor communication unit 39 configured to communicate with the pressure sensor signal processor 9, and a motor drive unit communication unit 37. The operation setting unit 31 includes operation switches 35a to 35e and a display panel 34. Furthermore, in the pressure sensor signal processing unit 9 includes a receiving communication unit 42 that receives gripping pressure values from the pressure sensors 51 and a transmitting communication unit 43 that transmits gripping pressure values to the pressure sensor communication unit 39.

The gripping unit 12 depicted in FIG. 5A is a bipolar high-frequency treatment instrument configured in such a manner that two gripping members having conductors or electrodes open and close to grip living tissue and perform high-frequency treatment. One or more pressure sensors 51 configured to detect gripping force for gripping the living tissue are disposed on front and back surfaces (a gripping surface with which the living tissue comes into contact and a non-gripping surface) of each of these gripping members. The pressure sensors 51 may be uniformly disposed to the front and back surfaces of the gripping unit 12 or may be densely arranged mainly in a region of each gripping surface with which the living tissue comes into contact. The necessary number of pressure sensors 51 may be arranged while appropriately considering a shape, a gripping state, and others of each gripping member.

The gripping unit 12 outputs gripping pressure values (sensor output signals) from the pressure sensors 51 each suggesting a living tissue gripping situation (whether a gripping state is adequately maintained) to the robotic treatment instrument control unit 7.

These gripping pressure values from the pressure sensors 51 are acquired by the pressure sensor signal processing unit 9 in a time-sharing manner, i.e., acquired at predetermined time intervals while gripping, e.g., a living mucosal membrane by the gripping members. Moreover, when strain gauges are used as the pressure sensors 51, since the strain gauges detect slight deformation of the gripping unit, the strain gauges do not necessarily have to be provided on the inner surfaces of the gripping members, and they may be provided on the outer surfaces of the same. The gripping pressure values measured by the pressure sensors 51 are amplified in the pressure sensor signal processor 9 and input to the robotic treatment instrument control unit 7. Additionally, a value of each encoder is transmitted to the robotic treatment instrument control unit 7. It is to be noted that, in the plurality of pressure sensors, the pressure sensors 51 which are not in contact with the living tissue are also present. In this case, a lower limit threshold value is set in advance, and it can be determined that the pressure sensor 51 which detect the pressure value not greater than the threshold value is not gripping the living tissue, and the output pressure value can be canceled to invalidate the value.

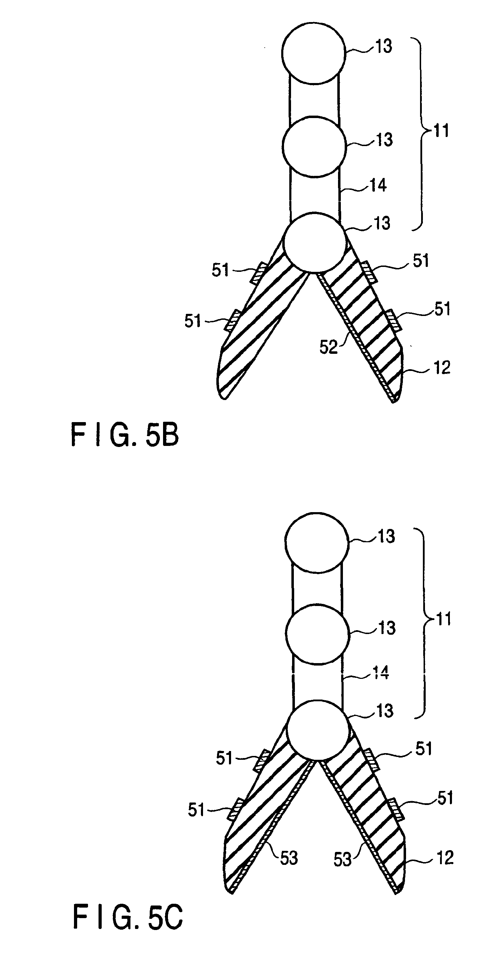

Further, FIGS. 5B and 5C are views showing first and second modifications of the gripping unit 12 in the second embodiment.

The first modification shown in FIG. 5B is a monopolar high-frequency treatment instrument. A gripping unit 12 of the treatment instrument the gripping unit opening and closing is formed of an insulator, and has a configuration provided an electrode portion 52 on a entire region of one gripping surface which may possibly come into contact with living tissue. In regard to pressure sensors 51, a plurality of pressure sensors 51 are arranged on outer sides of the gripping unit 12, i.e., non-gripping surfaces (which do not come into contact with living tissue).

The second modification shown in FIG. 5C is a bipolar high-frequency treatment instrument. A gripping unit 12 of the treatment instrument the gripping unit opening and closing is formed of an insulator, and has a configuration that provided electrode portions 53 are individually provided on entire regions of both gripping surfaces which may possibly come into contact with living tissue. In regard to pressure sensors 51, the plurality of pressure sensors 51 are arranged on outer sides like the first modification.

These first and second modifications can obtain the same functions and effects as those of the first embodiment. Moreover, since the electrode portions are provided on the entire surfaces of the gripping surfaces which come into contact with living tissue, contact can be assuredly achieved irrespective of a size of the living tissue.

Component parts and a flow of signals in the robot system for endoscopic treatment in the second embodiment will now be described with reference to FIG. 6. It is to be noted that the description is simplified in regard to parts equivalent to those in FIG. 2.

The master unit 6 outputs an instruction signal for the manipulator unit 11 and an instruction signal for the gripping unit 12 based on an operation of a surgeon to the robotic treatment instrument control unit 7. The robotic treatment instrument control unit 7 drives the corresponding motor 25 in the motor drive unit 21 in response to these instruction signals and thereby rotates the pivotally supported pulley 24. Based on this rotation, the wires 17 are pulled and paid out so that movement or a change in posture of the manipulator unit 11 coupled with the wires 17 or gripping or releasing of the gripping unit 12 coupled with the wires 17 is carried out.

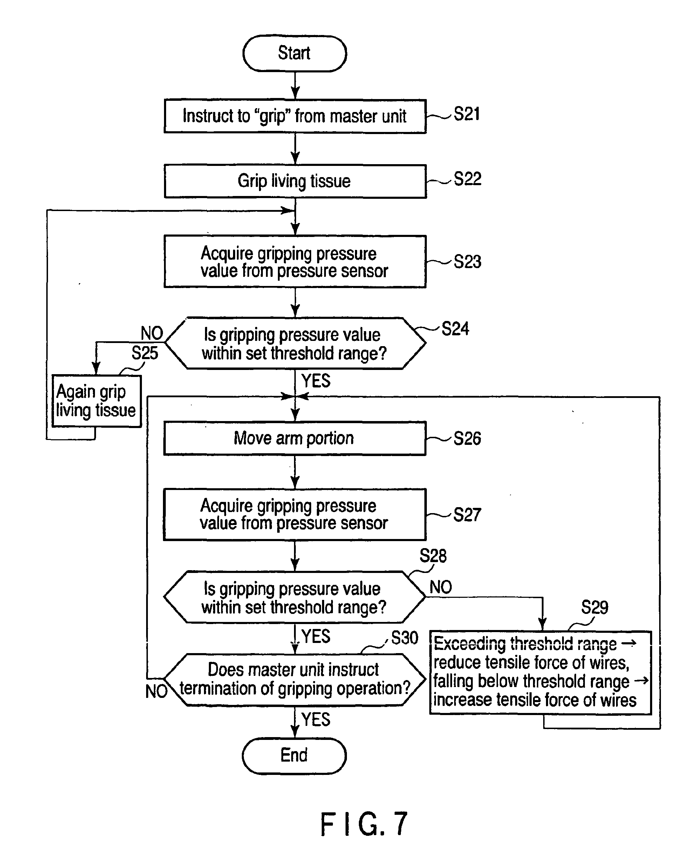

When the gripping unit 12 is in a gripping state, gripping pressure values from the pressure sensors 51 are acquired by the pressure sensor signal processor 9, subjected to signal processing, and then fed back to the robotic treatment instrument control unit 7. Based on such feedback control, the same gripping force in the gripping unit can be sustained, or the manipulator unit 11 can be maintained in the same posture (the gripping unit can be maintained at the same position). Such sustention will be described later in conjunction with FIG. 7.

As described above, at the same time as the actual gripping operation is performed, gripping pressure values detected by the pressure sensors 51 provided in the gripping unit 12 are subjected to signal processing in the pressure sensor signal processor 9, input to the robotic treatment instrument control unit 7, and compared with a threshold value having a predetermined given range (width). In this comparison, when the gripping pressure value from each pressure sensor 51 is within the threshold range, the gripping state based on the current wire tensile force is maintained. On the other hand, when the gripping pressure value is out of the threshold range, the wire tensile force is adjusted until this value falls within the threshold range.

Wire tensile control processing of the gripping unit 12 using the pressure sensors in the robot system for endoscopic treatment when the gripping portion is moved through the manipulator unit 11 will now be described with reference to a flowchart depicted in FIG. 7.

An operator (surgeon) operates the master unit 6 to instruct the robotic treatment instrument control unit 7 to grip desired living tissue by using the gripping unit 12 inserted in a body cavity (step S21). Based on this instruction, the robotic treatment instrument control unit 7 drives the corresponding motor 25 in the motor drive unit 21 and thereby rotates the pivotally supported pulley 24 to pull and pay out the wires 17 coupled with the pulley 24, thus gripping the living tissue by using the gripping unit 12 (step S22). At the time of gripping, the robotic treatment instrument control unit 7 acquires gripping pressure values from the pressure sensors 51 provided to the gripping unit 12 (step S23).

Then, the robotic treatment instrument control unit 7 compares each gripping pressure value with a predetermined threshold range and determines whether the gripping pressure value is within the threshold range (step S24). In this determination, when the gripping pressure value is out of the threshold range (NO), the robotic treatment instrument control unit 7 determines that the gripping unit 12 is not appropriately gripping the living tissue, operates the gripping unit 12 to again grip the living tissue (step S25), and returns the processing to step S23 to check the gripping pressure value. On the other hand, when the gripping pressure value is within the threshold range (YES), the robotic treatment instrument control unit 7 advances the processing to the next step.

At the time of performing treatment, for example, when living tissue in a gripped lesioned part is increased due to exfoliation, the manipulator unit 11 is driven to move the gripping unit 12 while the gripping unit 12 grips the living tissue in order to visually confirm a treatment region by the surgeon (step S26). At the time of this movement, the robotic treatment instrument control unit 7 acquires gripping pressure values from he pressure sensors 51 at predetermined time intervals (step S27), and occasionally determines whether the gripping pressure values are within the threshold range by comparing the gripping pressure values with the threshold range (step S28).

In this determination in step S28, when each gripping pressure value is out of the threshold range (NO), the robotic treatment instrument control unit 7 determines that the gripping state in the gripping unit 12 is no longer adequate. The robotic treatment instrument control unit 7 drives and controls the motor 25 to reduce wire tensile force when the gripping pressure value exceeds the threshold range in this determination and increase the wire tensile force when the gripping pressure value falls below the threshold range (step S29). On the other hand, if the gripping pressure value is within the threshold range (YES) in this determination in step S28, the robotic treatment instrument control unit 7 determines that the gripping unit 12 keeps normally gripping the living tissue and determines whether an instruction for termination (including temporary pause or suspension) of the gripping operation is issued from the master unit 6 in response to end of the treatment (step S30). When there is no instruction for termination of the gripping operation (NO), the robotic treatment instrument control unit 7 returns the processing to step S26 to continue movement of the gripping unit 12. On the other hand, when there is the instruction for termination of the gripping operation (YES), the robotic surgical instrument control unit 7 terminates the series of processing.

As described above, according to the robot system for endoscopic treatment of this embodiment, the pressure sensors are provided to the gripping unit of the robotic treatment instrument with the multi-joint structure, and control is effected to pay out or pull the wires based on a gripping pressure value obtained from each pressure sensor when the gripping unit is moved by drive of the manipulator unit until the gripping pressure value falls within the predetermined threshold range so that the normal gripping state in the gripping unit can be maintained. Based on this control, even if the gripping unit which is in the gripping state with the manipulator unit bending the joints is moved to a desired position, a change in gripping force due to a path length changing of the wires inserted inside or unevenness in gripping force due to friction between wires and coil pipes, resulting in gripping unit maintaining the normal gripping state for living tissue without producing slack or unnecessary pulling force in the wires.

Moreover, in the first embodiment according to the present invention, the description has been given as the robotic treatment instrument in the robot system for endoscopic treatment is the bipolar high-frequency treatment instrument as an example, the gripping unit having configuration with the bipolar high-frequency treatment instrument. When the robotic treatment instrument is used as the bipolar high-frequency surgical instrument, the gripping unit is formed by using an insulating member such as ceramics or a resin so that electrical short circuit can be prevented from occurring between the two gripping members. The electrodes are provided to the gripping units of these gripping members, high-frequency electrical power is applied to these electrodes at the time of gripping, and high-frequency surgery of sealing and cauterizing a part of a body cavity, a blood vessel, and others is performed. The robotic treatment instrument according to the first embodiment can be readily applied to the monopolar high-frequency treatment instrument. In this case, one of the two gripping members is formed of a conductor or an electrical insulating member (e.g., ceramics or zirconia) with an electrode provided thereto, and the other is formed of an electrical insulating member. A P-plate is attached to a patient to use the treatment instrument like a regular monopolar high-frequency treatment instrument. Of course, an instrument other than the P-plate may be used as long as it is an instrument which functions as an opposed electrode.

Also, in the second embodiment according to the present invention, the robotic treatment instrument in the robot system for endoscopic treatment can be readily configured to have a function of the bipolar high-frequency treatment instrument or the monopolar high-frequency treatment instrument. When providing the function of the bipolar high-frequency treatment instrument to the second embodiment, the pressure sensors provided to the gripping unit is prevented from being damaged due to heat generated by application of high-frequency power. Specifically, since the pressure sensors cannot be arranged on portions of the gripping unit that grip living tissue (the gripping surfaces), the pressure sensors should be provided on the non-gripping surfaces. Therefore, when strain gauges are used as the pressure sensors 51, since the strain gauges can detect slight deformation of the gripping unit, the strain gauges can be provided on the non-gripping surfaces. When applying to the monopolar high-frequency treatment instrument, like the first embodiment described above, one of the gripping members is formed of a conductor or an electrical insulating member with an electrode provided thereto, the other of the same can be formed of an electrical insulating member, and an instrument (e.g., a P-plate) that functions as an opposed electrode can be arranged.

### Explanation of Reference Symbols

1 - Controller, 2 - Robotic treatment instrument, 3 - Treatment instrument drive unit, 6 - Master unit, 6a - Operating portion, 7 - Robotic treatment instrument control unit, 8 - Tension sensor signal processor, 9 - Pressure sensor signal processor, 11 - Manipulator unit, 12 - Gripping unit, 13 - Joint portion, 14 - Rod portion, 15 - Soft sheath portion, 16 - External connecting portion, 17 - Wire, 21 - Motor drive unit, 22 - Wire coupling portion, 23 - Wire tension sensor, 24 - Pulley, 25 - Motor, 26 - Motor driver communication unit, 31 - Operation setting unit, 32 - CPU, 33 - Memory, 34 - Display panel, 35a to 35e - Operation switch, 36 - Motor driver, 37 - Motor driver communication unit, 38 - Tension sensor communication unit, 39 - Pressure sensor communication unit, 41, 44 - Cable, 42 - Receiving communication unit, 43 - Transmitting communication unit, 51 - Pressure sensor.

## Claims

1. A robot system for endoscopic treatment **characterized by** comprising:
a gripping unit (12) configured to be inserted into a body cavity along with an endoscopic apparatus wherein a desired living tissue is to be gripped by gripping members configured to move by a wire operation;
a manipulator unit (11) having at least one degree of freedom or more and including a multi-joint structure configured to bend at a desired angle or stretch wherein the gripping unit (12) is provided at a distal end;
a treatment instrument drive unit (3) configured to perform a pulling and a paying out of wires (17) for a gripping motion of the gripping unit (12) and moving a region of the multi-joint structure;
a gripping force detection unit configured to detect gripping force in the gripping members when the gripping unit (12) grips the living tissue; and
a control unit (1) configured to instruct the treatment instrument drive unit (3) on the wire operation of the gripping members so that the gripping force detected by the gripping force detection unit falls within a range of a predetermined threshold, when the gripping unit (12) in a gripping state is moved.

2. The robot system for the endoscopic treatment according to claim 1, **characterized in that**
the treatment instrument drive unit (3) is composed of:
pulleys (24) wherein one ends of each of pairs of wires (17) are coupled to each of the gripping members of the gripping unit (12) and the movable region of the multi-joint structure of the manipulator unit (11) and the other ends of each of the pairs of wires (17) are coupled to each of the pulleys (24); and
motors (25) wherein each of the motors (25) pivotally supports each of the pulleys (24) and is configured to drive by control of the control unit (1), and
the treatment instrument drive unit (3) is configured to open and close the gripping members and bend at the desired angle and stretch the movable region of the multi-joint structure by pulling and paying out the wires (17) accompanied with rotation of the pulleys (24).

3. The robot system for the endoscopic treatment according to claim 1, **characterized in that**
the gripping force detection unit is composed of a wire tension sensor (23) configured to detect the gripping force based on tensile force of the wire coupling to the gripping members of the gripping unit (12).

4. The robot system for the endoscopic treatment according to claim 1, **characterized in that**
the gripping force detection unit is composed of one pressure sensor (51) or more arranged at the gripping members of the gripping unit (12) and configured to output a gripping pressure value when the living tissue is gripped.

5. The robot system for the endoscopic treatment according to claim 1, **characterized in that**
the manipulator unit (11) is configured to freely bend at six degrees of freedom of XYZ directions, a rotating direction, a yawing direction and a pitch direction by coupling rod members (14) and joint members (13) with each other alternately.

6. The robot system for the endoscopic treatment according to claim 3, **characterized in that**
the robot system for the endoscopic treatment includes
electrodes (53) wherein each of the electrodes is provided with a gripping surface of each of the two gripping members, the two gripping members are formed of an insulating material and high-frequency power is to be applied to the electrodes when the living tissue is gripped, and
the robot system for the endoscopic treatment has a function as a bipolar high-frequency treatment instrument configured to perform a high-frequency treatment including sealing and cauterizing with respect to the living tissue.

7. The robot system for the endoscopic treatment according to claim 3, **characterized in that**
the robot system for the endoscopic treatment further comprises:
an electrode (52) provided on a gripping surface of one of the two gripping members wherein the two gripping members are formed of an insulating material and high-frequency power is to be applied to the electrode when the living tissue is gripped; and
an opposed electrode arranged near the electrode, and
the robot system for the endoscopic treatment has a function as a monopolar high-frequency treatment instrument configured to perform a high-frequency treatment including sealing and cauterizing with respect to the living tissue.

8. The robot system for the endoscopic treatment according to claim 4, **characterized in that**
the robot system for the endoscopic treatment further comprises
electrodes (53) wherein each of the electrodes is provided with a gripping surface of each of the two gripping members, the two gripping members are formed of an insulating material, the pressure sensor is arranged on a non-gripping surface of the gripping unit and high-frequency power is to be applied to the electrodes when the living tissue is gripped, and
the robot system for the endoscopic treatment has a function as a bipolar high-frequency treatment instrument configured to perform a high-frequency treatment including sealing and cauterizing with respect to the living tissue.

9. The robot system for the endoscopic treatment according to claim 4, **characterized in that**
the pressure sensor (51) is arranged on a non-gripping surface of the gripping unit (12),
the robot system for the endoscopic treatment is composed of:
an electrode (52) provided on a gripping surface of one of the two gripping members wherein the two gripping members are formed of an insulating material and high-frequency power is applied to the electrode when the living tissue is gripped; and
an opposed electrode arranged near the electrode, and
the robot system for the endoscopic treatment has a function as a monopolar high-frequency treatment instrument configured to perform a high-frequency treatment including sealing and cauterizing with respect to the living tissue.

10. The robot system for the endoscopic treatment according to claim 1, **characterized in that**
the robot system for the endoscopic treatment is a master/slave robot system comprising a master unit (6) which is an instruction input device for remotely operating the gripping unit (12) and the manipulator unit (11).

## Patentansprüche

1. Robotersystem zur endoskopischen Behandlung, **dadurch gekennzeichnet, dass** es umfasst:
eine Greifeinheit (12), die dazu ausgebildet ist, zusammen mit einem Endoskopiegerät in eine Körperöffnung eingeführt zu werden, wobei gewünschtes lebendes Gewebe durch Greifelemente zu greifen ist, die dazu ausgebildet sind, mittels Drahtbetrieb bewegt zu werden;
eine Manipulatoreinheit (11) mit mindestens einem Freiheitsgrad oder mehr und einer Mehrfachgelenkstruktur, die dazu ausgebildet ist, in einem gewünschten Winkel abgewinkelt oder gestreckt zu werden, wobei die Greifeinheit (12) an einem distalen Ende vorgesehen ist;
eine Behandlungsinstrumentantriebseinheit (3), die dazu ausgebildet ist, Drähte (17) anzuziehen und laufen zu lassen, um eine Greifbewegung der Greifeinheit (12) auszuführen und einen Bereich der Mehrfachgelenkstruktur zu bewegen;
eine Greifkrafterfassungseinheit, die dazu ausgebildet ist, Greifkraft in den Greifelementen zu erfassen, wenn die Greifeinheit (12) das lebende Gewebe greift; und
eine Steuereinheit (1), die dazu ausgebildet ist, die Behandlungsinstrumentantriebseinheit (3) bezüglich des Drahtbetriebs der Greifelemente so zu instruieren, dass die von der Greifkrafterfassungseinheit erfasste Greifkraft in einen Bereich eines vorbestimmten Schwellenwerts fällt, wenn die Greifeinheit (12) in einem Greifzustand bewegt wird.

2. Robotersystem zur endoskopischen Behandlung nach Anspruch 1, **dadurch gekennzeichnet, dass**
die Behandlungsinstrumentantriebseinheit (3) aus folgenden Komponenten besteht:
Rollen (24), wobei die einen Enden jedes Drahtpaars (17) mit jedem der Greifelemente der Greifeinheit (12) und dem beweglichen Bereich der Mehrfachgelenkstruktur der Manipulatoreinheit (11) gekoppelt sind und die anderen Enden jedes Drahtpaars (17) mit jeder der Rollen (24) gekoppelt sind; und
Motoren (25), wobei jeder der Motoren (25) jede der Rollen (24) schwenkbar hält und zum Antrieb unter Steuerung der Steuereinheit (1) ausgebildet ist, und
die Behandlungsinstrumentantriebseinheit (3) dazu ausgebildet ist, die Greifelemente zu öffnen und zu schließen und den beweglichen Bereich der Mehrfachgelenkstruktur durch Anziehen und Laufenlassen der Drähte (17) zusammen mit einer Drehung der Rollen (24) im gewünschten Winkel abzuwinkeln und zu strecken.

3. Robotersystem zur endoskopischen Behandlung nach Anspruch 1, **dadurch gekennzeichnet, dass**
die Greifkrafterfassungseinheit aus einem Drahtzugsensor (23) besteht, der dazu ausgebildet ist, die Greifkraft auf der Basis einer Zugkraft der Drahtkopplung mit den Greifelementen der Greifeinheit (12) zu erfassen.

4. Robotersystem zur endoskopischen Behandlung nach Anspruch 1, **dadurch gekennzeichnet, dass**
die Greifkrafterfassungseinheit aus einem Drucksensor (51) oder mehr besteht, der/die an den Greifelementen der Greifeinheit (12) angeordnet ist/sind und dazu ausgebildet ist/sind, einen Greifdruckwert auszugeben, wenn das lebende Gewebe gegriffen wird.

5. Robotersystem zur endoskopischen Behandlung nach Anspruch 1, **dadurch gekennzeichnet, dass**
die Manipulatoreinheit (11) dazu ausgebildet ist, in sechs Freiheitsgraden in XYZ-Richtungen, eine Drehrichtung, eine Gierrichtung und eine Nickrichtung, durch abwechselnde Kopplung von Stangenelementen (14) und Verbindungselementen (13) frei abgewinkelt zu werden.

6. Robotersystem zur endoskopischen Behandlung nach Anspruch 3, **dadurch gekennzeichnet, dass**
das Robotersystem zur endoskopischen Behandlung umfasst:
Elektroden (53), wobei jede der Elektroden mit einer Greifoberfläche jedes der zwei Greifelemente versehen ist, wobei die zwei Greifelemente aus einem Isoliermaterial gebildet sind und Hochfrequenzstrom an die Elektroden anzulegen ist, wenn das lebende Gewebe gegriffen wird, und
das Robotersystem zur endoskopischen Behandlung eine Funktion als ein bipolares Hochfrequenzbehandlungsinstrument hat, das dazu ausgebildet ist, eine Hochfrequenzbehandlung durchzuführen, die Versiegeln und Kauterisieren hinsichtlich des lebenden Gewebes umfasst.

7. Robotersystem zur endoskopischen Behandlung nach Anspruch 3, **dadurch gekennzeichnet, dass**
das Robotersystem zur endoskopischen Behandlung ferner umfasst:
eine Elektrode (52), die auf einer Greifoberfläche eines der zwei Greifelemente vorgesehen ist, wobei die zwei Greifelemente aus einem Isoliermaterial gebildet sind und Hochfrequenzstrom an die Elektroden anzulegen ist, wenn das lebende Gewebe gegriffen wird, und
eine gegenüberliegende Elektrode in der Nähe der Elektrode angeordnet ist, und
das Robotersystem zur endoskopischen Behandlung eine Funktion als ein monopolares Hochfrequenzbehandlungsinstrument hat, das dazu ausgebildet ist, eine Hochfrequenzbehandlung durchzuführen, die Versiegeln und Kauterisieren hinsichtlich des lebenden Gewebes umfasst.

8. Robotersystem zur endoskopischen Behandlung nach Anspruch 4, **dadurch gekennzeichnet, dass**
das Robotersystem zur endoskopischen Behandlung ferner umfasst:
Elektroden (53), wobei jede der Elektroden mit einer Greifoberfläche jedes der zwei Greifelemente versehen ist, wobei die zwei Greifelemente aus einem Isoliermaterial gebildet sind, der Drucksensor auf einer nicht greifenden Oberfläche der Greifeinheit angeordnet ist und Hochfrequenzstrom an die Elektroden anzulegen ist, wenn das lebende Gewebe gegriffen wird, und
das Robotersystem zur endoskopischen Behandlung eine Funktion als ein bipolares Hochfrequenzbehandlungsinstrument hat, das dazu ausgebildet ist, eine Hochfrequenzbehandlung durchzuführen, die Versiegeln und Kauterisieren hinsichtlich des lebenden Gewebes umfasst.

9. Robotersystem zur endoskopischen Behandlung nach Anspruch 4, **dadurch gekennzeichnet, dass**
der Drucksensor (51) auf einer nicht greifenden Oberfläche der Greifeinheit (12) angeordnet ist,
das Robotersystem zur endoskopischen Behandlung aus folgenden Komponenten besteht:
einer Elektrode (52), die auf einer Greifoberfläche eines der zwei Greifelemente vorgesehen ist, wobei die zwei Greifelemente aus einem Isoliermaterial gebildet sind und Hochfrequenzstrom an die Elektrode angelegt wird, wenn das lebende Gewebe gegriffen wird, und
einer gegenüberliegenden Elektrode, die in der Nähe der Elektrode angeordnet ist, und
das Robotersystem zur endoskopischen Behandlung eine Funktion als ein monopolares Hochfrequenzbehandlungsinstrument hat, das dazu ausgebildet ist, eine Hochfrequenzbehandlung durchzuführen, die Versiegeln und Kauterisieren hinsichtlich des lebenden Gewebes umfasst.

10. Robotersystem zur endoskopischen Behandlung nach Anspruch 1, **dadurch gekennzeichnet, dass**
das Robotersystem zur endoskopischen Behandlung ein Master/Slave-Robotersystem ist, das eine Mastereinheit (6) umfasst, die eine Anweisungseingabevorrichtung zur ferngesteuerten Bedienung der Greifeinheit (12) und der Manipulatoreinheit (11) ist.

## Revendications

1. Système robotisé pour traitement endoscopique, **caractérisé en ce qu'**il comprend :
une unité d'agrippement (12) configurée pour être introduite dans une cavité corporelle ensemble avec un appareil endoscopique, de sorte de qu'un tissu vivant désiré doit être agrippé par des éléments d'agrippement configurés pour se déplacer par actionnement d'un fil ;
une unité de manipulation (11) ayant au moins un degré de liberté ou plus et incluant une structure à joints multiples configurée pour se cintrer sous un angle désiré ou s'étirer quand l'unité d'agrippement (12) est prévue à une extrémité distale ;
une unité d'entraînement (3) pour instrument de traitement, configurée pour exécuter une rétraction et un déploiement d'un fil (17) pour un mouvement d'agrippement de l'unité d'agrippement (12) et un déplacement d'une région de la structure à joints multiples ;
une unité de détection de force d'agrippement configurée pour détecter une force d'agrippement dans les éléments d'agrippement quand l'unité d'agrippement (12) agrippe les tissus vivants ; et
une unité de commande (1) configurée pour donner des instructions à l'unité d'entraînement (3) pour instrument de traitement lors de l'actionnement des éléments d'agrippement au moyen du fil de sorte que la force d'agrippement détectée par l'unité de détection de force d'agrippement tombe dans une plage d'un seuil prédéterminé quand l'unité d'agrippement (12) dans un état d'agrippement est déplacée.

2. Système robotisé pour traitement endoscopique selon la revendication 1, **caractérisé en ce que**
l'unité d'entraînement (3) pour instrument de traitement est composée :
de poulies (24), de sorte que des extrémités de chacun des fils d'une paire de fils (17) sont couplées à chacun des éléments d'agrippement de l'unité d'agrippement (12) et à la région mobile de la structure à joints multiples de l'unité de manipulation (11) et les autres extrémités de chacune des paires de fils (17) sont couplées à chacune des poulies (24) ; et
de moteurs (25), de sorte que chacun des moteurs (25) supporte en pivotement chacune des poulies (24) et est configuré pour entraîner par commande de l'unité de commande (1), et
l'unité d'entraînement (3) pour instrument de traitement est configurée pour ouvrir et fermer les éléments d'agrippement et se cintrer sous l'angle désiré et étirer la région mobile de la structure à joints multiples par rétraction et déploiement des fils (17), accompagné d'une rotation des poulies (24).

3. Système robotisé pour traitement endoscopique selon la revendication 1, **caractérisé en ce que**
l'unité de détection de force d'agrippement est composée d'un capteur de tension de fil (23) configuré pour détecter la force d'agrippement en se basant sur la force de traction du fil qui est couplé aux éléments d'agrippement de l'unité d'agrippement (12).

4. Système robotisé pour traitement endoscopique selon la revendication 1, **caractérisé en ce que**
l'unité de détection de force d'agrippement est composée d'un capteur de pression (51) ou plus, agencé au niveau des éléments d'agrippement de l'unité d'agrippement (12) et configuré pour délivrer une valeur de pression d'agrippement quand les tissus vivants sont agrippés.

5. Système robotisé pour traitement endoscopique selon la revendication 1, **caractérisé en ce que**
l'unité de manipulation (11) est configurée pour être librement cintrée à six degrés de liberté dans les directions XYZ, une direction de rotation, une direction en lacets et une direction en roulis en couplant des éléments en forme de tiges (14) et des éléments en forme de joints (13) alternativement les uns avec les autres.

6. Système robotisé pour traitement endoscopique selon la revendication 3, **caractérisé en ce que**
le système robotisé pour traitement endoscopique inclut
des électrodes (53), dans lesquelles chacune des électrodes est dotée d'une surface d'agrippement de chacun des deux éléments d'agrippement, les deux éléments d'agrippement sont formés en un matériau isolant et une puissance à haute fréquence doit être appliquée aux électrodes quand les tissus vivants sont agrippés, et
le système robotisé pour traitement endoscopique fonctionne comme un instrument de traitement bipolaire à haute fréquence configuré pour exécuter un traitement à haute fréquence incluant obturation et cautérisation vis-à-vis des tissus vivants.

7. Système robotisé pour traitement endoscopique selon la revendication 3, **caractérisé en ce que**
le système robotisé pour traitement endoscopique comprend encore :
une électrode (52) prévue sur une surface d'agrippement de l'un des deux éléments d'agrippement, les deux éléments d'agrippement étant formés d'un matériau isolant, et une puissance à haute fréquence doit être appliquée à l'électrode quand les tissus vivants sont agrippés ; et
une électrode opposée agencée à proximité de l'électrode, et
le système robotisé pour traitement endoscopique présente la fonction d'un instrument de traitement monopolaire à haute fréquence configuré pour exécuter un traitement à haute fréquence incluant obturation et cautérisation vis-à-vis des tissus vivants.

8. Système robotisé pour traitement endoscopique selon la revendication 4, **caractérisé en ce que**
le système robotisé pour traitement endoscopique comprend encore des électrodes (53), telles que chacune des électrodes est prévue avec une surface d'agrippement de chacun des deux éléments d'agrippement, les deux éléments d'agrippement sont formés en matériau isolant, le capteur de pression est agencé sur une surface non-agrippante de l'unité d'agrippement et une puissance à haute fréquence doit être appliquée aux électrodes quand les tissus vivants sont agrippés, et
le système robotisé pour traitement endoscopique présente la fonction d'un instrument de traitement bipolaire à haute fréquence configuré pour exécuter un traitement à haute fréquence incluant obturation et cautérisation vis-à-vis des tissus vivants.

9. Système robotisé pour traitement endoscopique selon la revendication 4, **caractérisé en ce que**
le capteur de pression (51) est agencé sur une surface non-agrippante de l'unité d'agrippement (12),
le système robotisé pour traitement endoscopique est composé :
d'une électrode (52) prévue sur une surface d'agrippement de l'un des deux éléments d'agrippement, les deux éléments d'agrippement étant formés d'un matériau isolant, et une puissance à haute fréquence est appliquée à l'électrode quand les tissus vivants sont agrippés ; et
d'une électrode opposée agencée à proximité de l'électrode, et
le système robotisé pour traitement endoscopique présente la fonction d'un instrument de traitement monopolaire à haute fréquence configuré pour exécuter un traitement à haute fréquence incluant obturation et cautérisation vis-à-vis des tissus vivants.

10. Système robotisé pour traitement endoscopique selon la revendication 1, **caractérisé en ce que**
le système robotisé pour traitement endoscopique est un système robotisé maître/esclave comprenant une unité maître (6) qui est un dispositif de saisie d'instructions pour actionner à distance l'unité d'agrippement (12) et l'unité de manipulation (11).
